(19) Europäisches Patentamt / European Patent Office / Office européen des brevets

(11) **EP 2 046 269 B1**

(12) **EUROPEAN PATENT SPECIFICATION**

(45) Date of publication and mention
of the grant of the patent:
**15.12.2010 Bulletin 2010/50**

(51) Int Cl.:
*A61K 8/11* (2006.01)        *A61Q 1/12* (2006.01)
*A61Q 5/02* (2006.01)        *B01J 13/00* (2006.01)
*C11D 3/50* (2006.01)        *C11D 17/00* (2006.01)

(21) Application number: **07811020.2**

(22) Date of filing: **01.08.2007**

(86) International application number:
**PCT/US2007/017268**

(87) International publication number:
**WO 2008/016684 (07.02.2008 Gazette 2008/06)**

(54) **BENEFIT AGENT CONTAINING DELIVERY PARTICLE**

PFLEGEMITTEL MIT FREISETZUNGSPARTIKEL

PARTICULE DE DISTRIBUTION CONTENANT UN AGENT BÉNÉFIQUE

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **01.08.2006 US 834670 P**

(43) Date of publication of application:
**15.04.2009 Bulletin 2009/16**

(60) Divisional application:
**10189079.6**

(73) Proprietor: **The Procter and Gamble Company
Cincinnati, OH 45202 (US)**

(72) Inventors:
• **CUNNINGHAM, Philip, Andrew
3080 Vossem (BE)**
• **DIHORA, Jiten, Odhavji
Hamilton, Ohio 45011 (US)**
• **LIU, Zaiyou
West Chester, Ohio 45069 (US)**
• **SANDS, Peggy, Dorothy
Appleton, Wisconsin 54915 (US)**
• **GUINEBRETIERE, Sandra, Jacqueline
Appleton, Wisconsin 54913 (US)**

(74) Representative: **Mather, Peter Geoffrey
NV Procter & Gamble Services Company SA
100 Temselaan
1853 Strombeek-Bever (BE)**

(56) References cited:
EP-A- 0 965 326        EP-A- 1 533 415
EP-A- 1 589 092        WO-A-99/55819
WO-A2-2007/143869      US-A1- 2005 003 975
US-A1- 2005 176 598    US-A1- 2006 128 586

**Description**

FIELD OF INVENTION

The present application relates to benefit agent containing delivery particles, compositions comprising such particles, and processes for making and using such particles and compositions.

BACKGROUND OF THE INVENTION

Benefit agents such as perfumes, insect repellants, decongestants, and pheromones, are expensive and generally less effective when employed at high levels in personal care compositions, cleaning compositions, and fabric care compositions. As a result, there is a desire to maximize their effectiveness. One method of achieving such objective is to improve their delivery efficiency. Unfortunately, it is difficult to improve the delivery efficiencies of such agents as they may be lost do to their physical or chemical characteristics. In an effort to improve their delivery efficiency, artisans have employed encapsulation technologies wherein one or more benefit agent is encapsulated, typically within a melamine formaldehyde and/or urea based shell. Unfortunately, such agents may still migrate through such shells at undesirable rates. Thus what needed are one or more benefit compositions that do not suffer such drawback.

SUMMARY OF THE INVENTION

The present invention relates to benefit agent containing delivery particles comprising a core material and a wall material that at least partially surrounds the core material. The present invention also relates to compositions comprising said particles, and processes for making and using such particles and compositions.

DETAILED DESCRIPTION OF THE INVENTION

Definitions

As used herein "consumer product" means baby care, beauty care, fabric & home care, family care, feminine care, health care, snack and/or beverage products or devices intended to be used or consumed in the form in which it is sold, and not intended for subsequent commercial manufacture or modification. Such products include but are not limited to diapers, bibs, wipes; products for and/or methods relating to treating hair (human, dog, and/or cat), including, bleaching, coloring, dyeing, conditioning, shampooing, styling; deodorants and antiperspirants; personal cleansing; cosmetics; skin care including application of creams, lotions, and other topically applied products for consumer use; and shaving products, products for and/or methods relating to treating fabrics, hard surfaces and any other surfaces in the area of fabric and home care, including: air care, car care, dishwashing, fabric conditioning (including softening), laundry detergency, laundry and rinse additive and/or care, hard surface cleaning and/or treatment, and other cleaning for consumer or institutional use; products and/or methods relating to bath tissue, facial tissue, paper handkerchiefs, and/or paper towels; tampons, feminine napkins; products and/or methods relating to oral care including toothpastes, tooth gels, tooth rinses, denture adhesives, tooth whitening; over-the-counter health care including cough and cold remedies, pain relievers, RX pharmaceuticals, pet health and nutrition, and water purification; processed food products intended primarily for consumption between customary meals or as a meal accompaniment (non-limiting examples include potato chips, tortilla chips, popcorn, pretzels, corn chips, cereal bars, vegetable chips or crisps, snack mixes, party mixes, multigrain chips, snack crackers, cheese snacks, pork rinds, corn snacks, pellet snacks, extruded snacks and bagel chips); and coffee.

As used herein, the term "cleaning composition" includes, unless otherwise indicated, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, including antibacterial hand-wash types, cleaning bars, mouthwashes, denture cleaners, dentifrice, car or carpet shampoos, bathroom cleaners; hair shampoos and hair-rinses; shower gels and foam baths and metal cleaners; as well as cleaning auxiliaries such as bleach additives and "stain-stick" or pre-treat types, substrate-laden products such as dryer added sheets, dry and wetted wipes and pads, nonwoven substrates, and sponges; as well as sprays and mists.

As used herein, the term "fabric care composition" includes, unless otherwise indicated, fabric softening compositions, fabric enhancing compositions, fabric freshening compositions and combinations there of.

As used herein, the phrase "benefit agent containing delivery particle" encompasses microcapsules including perfume microcapsules.

As used herein, the term "particle" is synonymous with the phrase "benefit agent containing delivery particle".

As used herein, the articles "a" and "an" when used in a claim, are understood to mean one or more of what is claimed or described.

As used herein, the terms "include", "includes" and "including" are meant to be non-limiting.

The test methods disclosed in the Test Methods Section of the present application must be used to determine the respective values of the parameters of Applicants' inventions.

Unless otherwise noted, all component or composition levels are in reference to the active portion of that component or composition, and are exclusive of impurities, for example, residual solvents or by-products, which may be present in commercially available sources of such components or compositions.

All percentages and ratios are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Benefit Agent Containing Delivery Particle

Applicants discovered that the problem of achieving effective and efficient benefit agent delivery can be solved in an economical manner when a benefit agent containing delivery particle comprising a benefit agent composition having a certain combination of physical and chemical characteristics is employed. Such physical and chemical characteristics are defined by the following parameters: ClogP, boiling point and in certain aspects, odor detection threshold.

In one aspect, a particle comprising a benefit agent composition and a wall material that surrounds said benefit agent composition, said benefit agent composition comprising a first material having a ClogP of from 2.5 to 6 and a boiling point of from 80 °C to less than 250 °C; and optionally, a second material having a ClogP of less than 2.5, or even less than 2 to about 0.1; and a ODT of less than about 100 ppb, from about 0.00001 ppb to about less than about 100 ppb, from about 0.00001 ppb to about less than about 50 ppb or even from about 0.00001 ppb to about less than about 20 ppb is disclosed.

In one aspect, said second material may have a boiling point of from about 80 °C to about 350 °C, from about 80 °C to about 3 10 °C, from about 80 °C to about 230 °C, or even from about 80 °C to about 150 °C.

In one aspect, said benefit agent composition comprises, based on total, benefit agent composition weight, from about 50%, 70%, 80%, 90% or even 100% of said first material.

In one aspect, the balance of said benefit agent composition may comprise said second material. In one aspect, said balance of said benefit agent composition may comprise, based on total balance weight, from about 10%, 20%, 30%, 40%, 50%, 60%, 70%, 80%, 90% or even 100% of said second material.

In one aspect, said benefit agent composition may comprise, based on total benefit agent composition weight, from about 50%, 70%, 80%, 90% or even 100% of said first material.

In one aspect, said first material may comprise one or more Table 1 materials and said second material may comprise one or more Table 2 materials.

In one aspect, said first material may comprise a perfume selected from the group consisting of Table 1 materials numbered 2, 3, 9, 10, 11, 12, 13, 15, 16, 22-30, 32, 33, 41, 42, 44, 45, 55-59 and 65 and combinations thereof, and said second maternal may comprise a perfume selected from the group consisting of Table 2 materials numbered 2, 3, 4, 6, 7, 9-14, 19 and combinations thereof.

In one aspect, Applicants' particle may have physical and chemical characteristics that are defined by the following parameters: particle size coefficient of variation, fracture strength, benefit agent retention ratio and average particle size. Such parameters may be combined to yield a Delivery Index. Thus, said particle may have a Delivery Index of at least about 0.05 at least about 7, at least about 70, or even from about 500 to about 2200.

In one aspect. Applicants' particle may comprise a core material and a wall material that surrounds the core material, said particle having:

 a.) a particle size coefficient of variation of from about 1.5 to about 6.0, from about 2.0 to about 3.5, or even from about 2.5 to about 3.2;

 b.) a fracture strength of from about 0,69 kPa (0.1 psia) to about 0,758 MPa (110 psia), from about 6,89 kPa (1 psia) to about 0,345 MPa (50 psia), or even from about 27,6 kPa (4 psia) to about 110 kPa (16 psia);

 c.) a benefit agent retention ratio of from about 2 to about 110, from about 30 to about 90, or even from about 40 to about 70; and

d.) an average particle size of from about 1 micron to about 100 microns, from about 5 microns to about 80 microns, or even from about 15 microns to about 50 microns.

In one aspect of Applicants' invention, said particle may have and/or comprise any combination of the parameters described in the present specification. In one aspect, useful wall materials include materials that are sufficiently impervious to the core material and the materials in the environment in which the benefit agent containing delivery particle will be employed, to permit the delivery benefit to be obtained. Suitable impervious wall materials are selected from the group of melamine cross-linked with formaldehyde. In one aspect, the wall material may comprise melamine cross-linked with formaldehyde.

Useful benefit agent compositions include perfumes, insect repellants, decongestants, and pheromones. Useful perfumes include the perfumes described

Table 1 Materials: Having ClogP of at least 2 and boiling point of less than about 280 °C

| Material No. | Class I Examples | Chemical Functionality |
| --- | --- | --- |
| 1 | 1,1-Dimethoxy-2,2,5-trimethyl-4-hexene | Acetal |
| 2 | Citronellol | Alcohol |
| 3 | Geraniol | Alcohol |
| 4 | cis-p-Menthan-7-ol (Mayol) | Alcohol |
| 5 | Nerol | Alcohol |
| 6 | Lavo Citronellol | Alcohol |
| 7 | (-)-Menthol | Alcohol |
| 8 | 4-Methyl-3-decen-5-ol | Alcohol |
| 9 | Dihydromyrcenol | Alcohol |
| 10 | Linalool | Alcohol |
| 11 | Tetrahydrolinalool | Alcohol |
| 12 | 2,6,10-Trimethyl-9-undecenal (Adoxal) | Aldehyde |
| 13 | Citral | Aldehyde |
| 14 | Cyclamen aldehyde | Aldehyde |
| 15 | alpha,alpha-Dimethyl-p-ethylphenylpropanal (Floralozone) | Aldehyde |
| 16 | 2,4-Dimethyl-3-cyclohexene-1-carboxaldehyde (Ligustral) | Aldehyde |
| 17 | 7-Formyl-5-isopropyl-2-methylbicyclo[2.2.2]oct-2-ene (Maceal) | Aldehyde |
| 18 | 2,6-Dimethyl-5-heptenal(Melonal) | Aldehyde |
| 19 | Methyl n-nonyl acetaldehyde | Aldehyde |
| 20 | 4-(1,1-Dimethylethyl)benzenepropanal(bourgeonal) | Aldehyde |
| 21 | Citronellal | Aldehyde |
| 22 | Decenal (Trans-4) | Aldehyde |
| 23 | Decyl Aldehyde | Aldehyde |
| 24 | Intreleven Aldehyde | Aldehyde |
| 25 | Lauric Aldehyde | Aldehyde |
| 26 | Nonyl Aldehyde | Aldehyde |
| 27 | Octyl Aldehyde | Aldehyde |
| 28 | Undecyl Aldehyde | Aldehyde |
| 29 | Undecylenic Aldehyde | Aldehyde |
| 30 | Forhydral | Aldehyde |

(continued)

| Material No. | Class I Examples | Chemical Functionality |
|---|---|---|
| 31 | 2,6-Dimethylheptyl-4 acetate | Ester |
| 32 | Allyl caproate | Ester |
| 33 | Allyl cyclohexanepropionate | Ester |
| 34 | Allyl heptanoate | Ester |
| 35 | Citronellyl acetate | Ester |
| 36 | Methyl 2-octynoate | Ester |
| 37 | beta-Methyl-gamma-octalactone | Ester |
| 38 | Methyl2-nonynoate | Ester |
| 39 | 2-tert.Butylcyclohexyl acetate | Ester |
| 40 | Tricyclodecenyl propionate / Frutene | Ester |
| 41 | Ethyl 2 methyl pentanoate | Ester |
| 42 | Methyl phenyl carbonyl acetate | Ester |
| 43 | Hexyl acetate | Ester |
| 44 | Eucalyptol | Ether |
| 45 | Rose oxide | Ether |
| 46 | 2,4-Dimethyl-4-phenyltetrahydrofuran | Ether |
| 47 | Rosyrane (2H-Pyran, 3,6-dihydro-4-methyl-2-phenyl-) | Ether |
| 48 | (-) alpha-Pinene | Hydrocarbon |
| 49 | (3E,5Z)-1,3,5-Undecatriene (galbanolene) | Hydrocarbon |
| 50 | Ethyl hexyl ketone | Ketone |
| 51 | Methyl nonyl ketone | Ketone |
| 52 | 2-Heptylcyclopentanone | Ketone |
| 53 | Isomenthone | Ketone |
| 54 | Menthone | Ketone |
| 55 | Damascenone | Ketone |
| 56 | Ionone gamma methyl | Ketone |
| 57 | Ionone alpha | Ketone |
| 58 | Ionone beta | Ketone |
| 59 | Delta Damascone | Ketone |
| 60 | gamma-Nonalactone | Lactone |
| 61 | gamma-Undecalactone | Lactone |
| 62 | gamma-Undecalactone | Lactone |
| 63 | Citronellyl nitrile | Nitrile |
| 64 | 3,7-Dimethyl-2(3),6-nonadienenitriles | Nitrile |
| 65 | 5-Methyl-3-heptanone oxime (stemone) | Oxime |
| 66 | Dihydroeugenol | Phenol |
| 67 | Thymol | Phenol |

Table 2 Materials: Materials having a ClogP of less than 2.5 and an ODT* of less than about 100 ppb

| Material No. | Material | Chemical Functionality |
|---|---|---|
| 1 | Cinnamic alcohol | Alcohol |
| 2 | Maltol | Alcohol |
| 3 | Hexenol; 3-Hexen-1-ol; cis-3-Hexenol;(Z)-3-Hexenol; | Alcohol |
| 4 | ethyl vanillin | Aldehyde |
| 5 | Heliotropin | Aldehyde |
| 6 | trans-2-heptenal | Aldehyde |
| 7 | vanillin | Aldehyde |
| 8 | Phenylacetaldehyde; | Aldehyde |
| 9 | Methyl anthranilate | Amine |
| 10 | Allyl cyclohexyloxyacetate (cyclo galbanate) | Ester |
| 11 | coumarin | Ester |
| 12 | Ethyl 2 methyl butyrate | Ester |
| 13 | Cis 3 hexenyl actetate | Ester |
| 14 | Glycolic acid, 2-pentyloxy-, allyl ester (AAG) | Ester |
| 15 | Methyl salicylate; | Ester |
| 16 | Flor Acetate | Ester |
| 17 | Methyl phenethyl ether (Keone) | Ester |
| 18 | Octanone; 3-Octanone; (ethyl amyl ketone) | Ketone |
| 19 | Raspberry Ketone; 4-(p-Hydroxyphenyl)-2-butanone; Oxanone, | Ketone |
| 20 | Cresol; 4-methylphenol; p-Cresol; | Phenol |
| 21 | eugenol | Phenol |
| 22 | Ethyl 3-methylthiopropionate; | Sulfide |
| * Per Standardized Human Olfactory Thresholds, W. Devos, F. Patte, J. Rouault, P. Laffort (France), and L.J. Van Gemert TNO-CIVO Food Analysisi Institute, P.O. Box 360, 3700 AJ Zeist (The Netherlands), IRL Press 1990. | | |

The perfume raw materials and accords may be obtained from one or more of the following companies Firmenich (Geneva, Switzerland), Givaudan (Argenteuil, France), IFF (Hazlet, NJ), Quest (Mount Olive, NJ), Bedoukian (Danbury, CT), Sigma Aldrich (St. Louis, MO), Millennium Specialty Chemicals (Olympia Fields, IL), Polarone International (Jersey City, NJ), Fragrance Resources (Keyport, NJ), and Aroma & Flavor Specialties (Danbury, CT).

Process of Making Benefit Agent Containing Delivery Particles

The particle disclosed in the present application may be made via the teachings of USPs 6,592,990; 5,188,753; 6,951,836; and 5,441,660 and the examples disclosed there in.

Anionic emulsifiers are typically used during the capsule making process to emulsify the benefit agent prior to microcapsule formation. While not being bound by theory, it is believed that the anionic materials adversely interact with the cationic surfactant actives that are often found in compositions such as fabric care compositions - this may yield an aesthetically unpleasing aggregation of particles that are employed in said composition. In addition to the unacceptable aesthetics, such aggregates may result in rapid phase separation of the particles from the bulk phase. Applicants discovered that such aggregates can be prevented by the addition of certain aggregate inhibiting materials including materials selected from the group consisting of salts, polymers and mixtures thereof. Useful aggregate inhibiting materials include, divalent salts such as magnesium salts, for example, magnesium chloride, magnesium acetate, magnesium phosphate, magnesium formate, magnesium boride, magnesium titanate, magnesium sulfate heptahydrate; calcium salts, for example, calcium chloride, calcium formate, calcium calcium acetate, calcium bromide; trivalent salts, such as aluminum salts, for example, aluminum sulfate, aluminum phosphate, aluminum chloride n-hydrate and polymers that have the ability to suspend anionic particles such as soil suspension polymers, for example, (polyethylene imines, alkoxylated polyethylene imines, polyquaternium-6 and polyquatemium-7.

In one aspect of the invention, the particles are manufactured by following the procedure described in USP 6,592,990 and are subsequently coated with a material to reduce the rate of leakage of the benefit agent from the particles when the particles are subjected to a bulk environment containing, for example, surfactants, polymers, and solvents. Non-limiting examples of coating materials that can serve as barrier materials include materials selected from the group consisting of polyvinyl pyrrolidone homopolymer, and its various copolymers with styrene, vinyl acetate, imidazole, primary and secondary amine containing monomers, methyl acrylate, polyvinyl acetal, maleic anhydride; polyvinyl alcohol homopolymer, and its various copolymers with vinyl acetate, 2-acrylamide-2-methylpropane sulfonate, primary and secondary amine containing monomers, imidazoles, methyl acrylate; polyacrylamides; polyacrylic acids; microcrystalline waxes; paraffin waxes; modified polysaccharides such as waxy maize or dent corn starch, octenyl succinated starches, derivatized starches such as hydroxyethylated or hydroxypropylated starches, carrageenan, guar gum, pectin, xanthan gum; modified celluloses such as hydrolyzed cellulose acetate, hydroxy propyl cellulose, methyl cellulose, and the like; modified proteins such as gelatin; hydrogenated and non-hydrogenated polyalkenes; fatty acids; hardened shells such as urea crosslinked with formaldehyde, gelatin-polyphosphate, melamine-formaldehyde, polyvinyl alcohol crosslinked with sodium tetraborate or gluteraldehyde; latexes of styrene-butadiene, ethyl cellulose, inorganic materials such as clays including magnesium silicates, aluminosilicates; sodium silicates, and the like; and mixtures thereof. Such materials can be obtained from CP Kelco Corp. of San Diego, California, USA; Degussa AG or Dusseldorf, Germany; BASF AG of Ludwigshafen, Germany; Rhodia Corp. of Cranbury, New Jersey, USA; Baker Hughes Corp. of Houston, Texas, USA; Hercules Corp. of Wilmington, Delaware, USA; Agrium Inc. of Calgary, Alberta, Canada, ISP of New Jeresy U.S.A.. In one aspect wherein the particle is employed in a fabric conditioning composition, the coating material may comprise sodium silicate. While not being bound by theory, it is believed that sodium silicate's solubility at high pH, but poor solubility at low pH makes it an ideal material for use on particles that may be used in compositions that are formulated at pH below 7 but used in an environment wherein the pH is greater or equal to 7.

Suitable equipment for use in the processes disclosed herein may include continuous stirred tank reactors, homogenizers, turbine agitators, recirculating pumps, paddle mixers, ploughshear mixers, ribbon blenders, vertical axis granulators and drum mixers, both in batch and, where available, in continuous process configurations, spray dryers, and extruders. Such equipment can be obtained from Lodige GmbH (Paderborn, Germany), Littleford Day, Inc. (Florence, Kentucky, U.S.A.), Forberg AS (Larvik, Norway), Glatt Ingenieurtechnik GmbH (Weimar, Germany), Niro (Soeborg, Denmark), Hosokawa Bepex Corp. (Minneapolis, Minnesota, USA), Arde Barinco (New Jersey, USA).

Compositions Comprising Benefit Agent Containing Delivery Particles

Applicants' compositions may comprise an embodiment of the particle disclosed in the present application. In one aspect, said composition is a consumer product. While the precise level of particle that is employed depends on the type and end use of the composition, a composition may comprise from about 0.01 to about 10, from about 0.1 to about 10, or even from about 0.2 to about 5 weight % of said particle based on total composition weight. In one aspect, a cleaning composition may comprise, from about 0.1 to about 1 weight % of such particle based on total cleaning composition weight of such particle. In one aspect, a fabric treatment composition may comprise, based on total fabric treatment composition weight, form about 0.01 to about 10% of such particle.

Aspects of the invention include the use of the particles of the present invention in laundry detergent compositions (e.g., TIDE™), hard surface cleaners (e.g., MR CLEAN™), automatic dishwashing liquids (e.g., CASCADE™), dishwashing liquids (e.g., DAWN™), and floor cleaners (e.g., SWIFFER™). Non-limiting examples of cleaning compositions may include those described in U.S. Pat. Nos. 4,515,705; 4,537,706; 4,537,707; 4,550,862; 4,561,998; 4,597,898; 4,968,451; 5,565,145; 5,929,022; 6,294,514; and 6,376,445. The cleaning compositions disclosed herein are typically formulated such that, during use in aqueous cleaning operations, the wash water will have a pH of between about 6.5 and about 12, or between about 7.5 and 10.5. Liquid dishwashing product formulations typically have a pH between about 6.8 and about 9.0. Cleaning products are typically formulated to have a pH of from about 7 to about 12. Techniques for controlling pH at recommended usage levels include the use of buffers, alkalis, acids, etc., and are well known to those skilled in the art.

Fabric treatment compositions disclosed herein may comprise a fabric softening active ("FSA"). Suitable fabric softening actives, include, but are not limited to, materials selected from the group consisting of quats, amines, fatty esters, sucrose esters, silicones, dispersible polyolefins, clays, polysaccharides, fatty oils, polymer latexes and mixtures thereof. Suitable FSAs are described in U.S. Pat. Pub. No. 2004/0204337 A1; 2004/0229769 A1; 2004/0204337 A1; 2002/0077265 A1; 2004/0142841 A1; 2003/0216274 A1; 2004/0038851 A1; 2004/0065208 A1 and 2005/0020476 A1 and in U.S. Patents 3,862,058; 3,948,790; 3,954,632; 4,062,647; 5,759,990 and 6,494,920, and publications, WO 02/18451; WO 06/007911 A1 and WO 06/007899 A1. Another class of optional fabric care actives is softening oils, which include but are not limited to, vegetable oils (such as soybean, sunflower, and canola), hydrocarbon based oils (natural and synthetic petroleum lubricants, polyolefins, isoparaffins, and cyclic paraffins), triolein, fatty esters, fatty alcohols, fatty amines, fatty amides, and fatty ester amines. Oils can be combined with fatty acid softening agents, clays, and silicones.

Adjunct Materials

While not essential for the purposes of the present invention, the non-limiting list of adjuncts illustrated hereinafter are suitable for use in the instant compositions and may be desirably incorporated in certain embodiments of the invention, for example to assist or enhance performance, for treatment of the substrate to be cleaned, or to modify the aesthetics of the composition as is the case with perfumes, colorants, dyes or the like. It is understood that such adjuncts are in addition to the components that are supplied via Applicants' delivery particles and FSAs. The precise nature of these additional components, and levels of incorporation thereof, will depend on the physical form of the composition and the nature of the operation for which it is to be used. Suitable adjunct materials include, but are not limited to, surfactants, builders, chelating agents, dye transfer inhibiting agents, dispersants, enzymes, and enzyme stabilizers, catalytic materials, bleach activators, polymeric dispersing agents, clay soil removal/antiredeposition agents, brighteners, suds suppressors, dyes, additional perfume and perfume delivery systems, structure elasticizing agents, fabric softeners, carriers, hydrotropes, processing aids and/or pigments. In addition to the disclosure below, suitable examples of such other adjuncts and levels of use are found in U.S. Patent Nos. 5,576,282, 6,306,812 B1 and 6,326,348 B1 that are incorporated by reference.

As stated, the adjunct ingredients are not essential to Applicants' cleaning and fabric care compositions. Thus, certain embodiments of Applicants' compositions do not contain one or more of the following adjuncts materials: bleach activators, surfactants, builders, chelating agents, dye transfer inhibiting agents, dispersants, enzymes, and enzyme stabilizers, catalytic metal complexes, polymeric dispersing agents, clay and soil removal/anti-redeposition agents, brighteners, suds suppressors, dyes, additional perfumes and perfume delivery systems, structure elasticizing agents, fabric softeners, carriers, hydrotropes, processing aids and/or pigments. However, when one or more adjuncts is present, such one or more adjuncts may be present as detailed below:

Surfactants - The compositions according to the present invention may comprise a surfactant or surfactant system wherein the surfactant can be selected from nonionic and/or anionic and/or cationic surfactants and/or ampholytic and/or zwitterionic and/or semi-polar nonionic surfactants. The surfactant is typically present at a level of from about 0.1%, from about 1%, or even from about 5% by weight of the cleaning compositions to about 99.9%, to about 80%, to about 35%, or even to about 30% by weight of the cleaning compositions.

Builders - The compositions of the present invention may comprise one or more detergent builders or builder systems. When present, the compositions may comprise at least about 1% builder, or from about 5% or 10% to about 80%, 50%, or even 30% by weight, of said builder. Builders include, but are not limited to, the alkali metal, ammonium and alkanolammonium salts of polyphosphates, alkali metal silicates, alkaline earth and alkali metal carbonates, aluminosilicate builders polycarboxylate compounds. ether hydroxypolycarboxylates, copolymers of maleic anhydride with ethylene or vinyl methyl ether, 1,3,5-trihydroxybenzene-2,4,6-trisulphonic acid, and carboxymethyl-oxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, oxydisuccinic acid, polymaleic acid, benzene 1,3,5-tricarboxylic acid, carboxymethyloxysuccinic acid, and soluble salts thereof.

Chelating Agents - The compositions herein may also optionally contain one or more copper, iron and/or manganese chelating agents. If utilized, chelating agents may comprise from about 0.1% by weight of the compositions herein to about 15%, or even from about 3.0% to about 15% by weight of the compositions herein.

Dye Transfer Inhibiting Agents - The compositions of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof When present in the compositions herein, the dye transfer inhibiting agents are present at levels from about 0.0001%, from about 0.01%, from about 0.05% by weight of the cleaning compositions to about 10%, about 2%, or even about 1% by weight of the cleaning compositions.

Dispersants - The compositions of the present invention can also contain dispersants. Suitable water-soluble organic materials are the homo- or co-polymeric acids or their salts, in which the polycarboxylic acid may comprise at least two carboxyl radicals separated from each other by not more than two carbon atoms.

Enzymes - The compositions may comprise one or more detergent enzymes which provide cleaning performance and/or fabric care benefits. Examples of suitable enzymes include, but are not limited to, hemicellulases, peroxidases, proteases, cellulases, xylanases, lipases, phospholipases, esterases, cutinases, pectinases, keratanases, reductases, oxidases, phenoloxidases, lipoxygenases, ligninases, pullulanases, tannases, pentosanases, malanases, β-glucanases, arabinosidases, hyaluronidase, chondroitinase, laccase, and amylases, or mixtures thereof. A typical combination is a cocktail of conventional applicable enzymes like protease, lipase, cutinase and/or cellulase in conjunction with amylase.

Enzyme Stabilizers - Enzymes for use in compositions, for example, detergents can be stabilized by various techniques. The enzymes employed herein can be stabilized by the presence of water-soluble sources of calcium and/or magnesium ions in the finished compositions that provide such ions to the enzymes.

Catalytic Metal Complexes - Applicants' compositions may include catalytic metal complexes. One type of metal-containing bleach catalyst is a catalyst system comprising a transition metal cation of defined bleach catalytic activity, such as copper, iron, titanium, ruthenium, tungsten, molybdenum, or manganese cations, an auxiliary metal cation having little or no bleach catalytic activity, such as zinc or aluminum cations, and a sequestrate having defined stability constants for the catalytic and auxiliary metal cations, particularly ethylenediaminetetraacetic acid, ethylenediaminetetra (methyl-enephosphonic acid) and water-soluble salts thereof. Such catalysts are disclosed in U.S. patent 4,430,243.

If desired, the compositions herein can be catalyzed by means of a manganese compound. Such compounds and levels of use are well known in the art and include, for example, the manganese-based catalysts disclosed in U.S. patent 5,576,282.

Cobalt bleach catalysts useful herein are known, and are described, for example, in U.S. patents 5,597,936 and 5,595,967. Such cobalt catalysts are readily prepared by known procedures, such as taught for example in U.S. patents 5,597,936, and 5,595,967.

Compositions herein may also suitably include a transition metal complex of a macropolycyclic rigid ligand - abreviated as "MRL". As a practical matter, and not by way of limitation, the compositions and cleaning processes herein can be adjusted to provide on the order of at least one part per hundred million of the benefit agent MRL species in the aqueous washing medium, and may provide from about 0.005 ppm to about 25 ppm, from about 0.05 ppm to about 10 ppm, or even from about 0.1 ppm to about 5 ppm, of the MRL in the wash liquor.

Preferred transition-metals in the instant transition-metal bleach catalyst include manganese, iron and chromium. Preferred MRL's herein are a special type of ultra-rigid ligand that is cross-bridged such as 5,12-diethyl-1,5,8,12-tetraazabicyclo[6.6.2]hexa-decane.

Suitable transition metal MRLs are readily prepared by known procedures, such as taught for example in WO 00/32601, and U.S. patent 6,225,464.

Processes of Making and Using Compositions

The compositions of the present invention can be formulated into any suitable form and prepared by any process chosen by the formulator, non-limiting examples of which are described in U.S. 5,879,584; U.S. 5,691,297; U.S. 5,574,005; U.S. 5,569,645; U.S. 5,565,422; U.S. 5,516,448; U.S. 5,489,392; and U.S. 5,486,303.

Method of Use

The benefit agent delivery particle disclosed herein and/or compositions containing the benefit agent delivery particle disclosed herein can be used to clean or treat a situs *inter alia* a surface or fabric. Typically at least a portion of the situs is contacted with an embodiment of Applicants' particle and/or such a composition, in neat form or diluted in a liquor, for example, a wash liquor and then the situs may be optionally washed and/or rinsed. In one aspect, a situs is optionally washed and/or rinsed, contacted with a particle according to the present invention or composition comprising said particle and then optionally washed and/or rinsed. For purposes of the present invention, washing includes but is not limited to, scrubbing, and mechanical agitation. The fabric may comprise most any fabric capable of being laundered or treated in normal consumer use conditions. Liquors that may comprise the disclosed compositions may have a pH of from about 3 to about 11.5. Such compositions are typically employed at concentrations of from about 500 ppm to about 15,000 ppm in solution. When the wash solvent is water, the water temperature typically ranges from about 5 °C to about 90 °C and, when the situs comprises a fabric, the water to fabric ratio is typically from about 1:1 to about 30:1.

TEST METHODS

It is understood that the test methods that are disclosed in the Test Methods Section of the present application must be used to determine the respective values of the parameters of Applicants' invention as such invention is described and claimed herein.

(1) <u>**Particle size distribution**</u>

a.) Place 1 gram of particles in 1 liter of distilled deionized (DI) water.

b.) Permit the particles to remain in the DI water for 10 minutes and then recover the particles by filtration.

c.) Determine the particle size distribution of the particle sample by measuring the particle size of 50 individual particles using the experimental apparatus and method of Zhang, Z.; Sun, G; "Mechanical Properties of Melamine-Formaldehyde microcapsules," J. Microencapsulation, vol 18, no. 5, pages 593-602, 2001.

d.) Average the 50 independent particle diameter measurements to obtain an average particle diameter.

e.) Use the 50 independent measurements to calculate a standard deviation of particle size using the following equation:

$$\mu = \sqrt{\frac{\sum (d - s)^2}{n - 1}}$$

where

$\mu$ is the standard deviation

s is the average particle diameter

d is the independent particle diameter

n is the total number of particles whose diameter is measured.

(2) <u>**Benefit Agent Retention Ratio**</u>

a.) Add 1 gram of particle to 99 grams of composition that the particle will be employed in.

b.) Age the particle containing composition of a.) above for 2 weeks at 40°C in a sealed, glass jar.

c.) Recover the particles from b.) above by filtration.

d.) Treat the particles of c.) above with a solvent that will extract all the benefit agent from the particles.

e.) Inject the benefit agent containing solvent from d.) above into a Gas Chromatograph and integrate the peak areas to determine the total quantity of benefit agent extracted from the particle sample.

f.) This quantity is then divided by the quantity that would be present if nothing had leaked out of the microcapsule (e.g. the total quantity of core material that is dosed into the composition via the microcapsules). This value is then multiplied by the ratio of average particle diameter to average particle thickness to obtain a Benefit Agent Retention Ratio.

A detailed analytical procedure to measure the Benefit Agent Retention Ratio is:

ISTD Solution

1. Weigh out 25mg dodecane into a weigh boat.

2. Rinse the dodecane into a 1000mL volumetric flask using ethanol.

3. Add ethanol to volume mark.

4. Stir solution until mixed. This solution is stable for 2 months.

Calibration Standard

1. Weigh out 75mg of core material into a 100 mL volumetric flask.

2. Dilute to volume with ISTD solution to from above. This standard solution is stable for 2 months.

3. Mix well.

4. Analyze via GC/FID.

Basic Sample Prep
(Prepare samples in triplicate)

1. Weigh 1.000 gram sample of aged composition containing particles into a 100 mL tri-pour beaker. Record weight.
2. Add 4 drops (approximately 0.1 gram) 2-ethyl-1,3-Hexanediol into the tri-pour beaker.
3. Add 50 mL Deionized water to the beaker. Stir for 1 minute.
4. Using a 60cc syringe, filter through a Millipore Nitrocellulose Filter Membrane (1.2 micron, 25 mm diameter).
5. Rinse through the filter with 10 mL of Hexane
6. Carefully remove the filter membrane and transfer to a 20 mL scintillation vial (using tweezers).
7. Add 10mL ISTD solution (as prepared above) to the scintillation vial containing the filter.
8. Cap tightly, mix, and heat vial at 60°C for 30min.
9. Cool to room temperature.
10. Remove 1mL and filter through a 0.45-micron PTFE syringe filter into GC vial. Several PTFE filters may be required to filter a 1mL sample aliquot.
11. Analyze via GC/FID.

GG/FID Analysis Method:

Column - 30m X 0.25mm id, 1-um DB-1 phase
GC - 6890 GC equipped with EPC control and constant flow capability
Method - 50°C, 1min. hold, temperature ramp of 4°C/min. to 300°C, and hold for 10min.
Injector - 1uL splitless injection at 240°C

GC/FID Analysis Method - Microbore Column Method:

Column - 20m X 0.1mm id, 0.1 $\mu$m DB-5
GC - 6890 GC equipped with EPC control and constant flow capability (constant flow 0.4mL/min)
Method - 50°C, no hold, temperature ramp of 16°C/min to 275°C, and hold for 3min.
Injector - 1$\mu$L split injection (80:1 split) at 250°C

Calculations:

$$\% \; Total \; Perfume = \frac{A_{IS} \; x \; W_{per\text{-}std} \; x \; A_{per\text{-}sam}}{A_{per\text{-}std} \; x \; A_{is\text{-}sam} \; x \; W_{sam}} \; x \; 100\%$$

where

$A_{is}$ = Area of internal standard in the core material calibration standard;
$W_{per\text{-}std}$ = weight of core material in the calibration sample
$A_{per\text{-}sam}$ = Area of core material peaks in the composition containing particle sample;
$A_{per\text{-}std}$ = Area of core material peaks in the calibration sample.
$A_{is\text{-}sam}$ = Area of internal standard in composition containing particle sample;
$W_{sam}$ = Weight of the composition containing particle sample

$$Re\mathit{tention\_Ratio} = \left( \frac{Total\_Perfume}{Perfume\_Dosed\_Into\_Pr\mathit{oduct}\_Via\_Microcapsules} \right)\left( \frac{\mu}{T} \right)$$

where
$\mu$ is the average particle diameter, from Test Method 1
T is the average particle thickness as calculated from Test Method 3

(3) **Fracture Strength**

a.) Place 1 gram of particles in 1 liter of distilled deionized (DI) water.

b.) Permit the particles to remain in the DI water for 10 minutes and then recover the particles by filtration.

c.) Determine the average rupture force of the particles by averaging the rupture force of 50 individual particles. The rupture force of a particle is determined using the procedure given in Zhang, Z.; Sun, G; "Mechanical Properties of Melamine-Formaldehyde microcapsules," J. Microencapsulation, vol 18, no. 5, pages 593-602, 2001. Then calculate the average fracture pressure by dividing the average rupture force (in Newtons) by the average cross-sectional area (as determined by Test Method I above) of the spherical particle ($\pi r^2$, where r is the radius of the particle before compression).

d.) Calculate the average fracture strength by using the following equation:

$$\sigma_{fracture\_stress} = \frac{P}{4(d/T)}$$

where

$P$ is the average fracture pressure, calculated by taking the average of individual microcapsule force measurements (each independent measurements yields a fracture force. This force is divided by the cross sectional area of the microcapsule)

$d$ is the average diameter of the particle (as determined by Test Method 1 above)

$T$ is the average shell thickness of the particle shell as determined by the following equation:

$$T = \frac{r_{capsule}(1-c)\rho_{perfume}}{3[c\rho_{wall} + (1-c)\rho_{perfume}]}$$

where

$c$ is the average perfume content in the particle

$r$ is the average particle radius

$\rho_{wall}$ is the average density of the shell as determined by ASTM method B923-02, "Standard Test Method for Metal Powder Skeletal Density by Helium or Nitrogen Pycnometry", ASTM International.

$\rho_{perfume}$ is the average density of the perfume as determined by ASTM method D1480-93(1997) "Standard Test Method for Density and Relative Density (Specific Gravity) of Viscous Materials by Bingham Pycnometer", ASTM International.

**(4) ClogP**

The "calculated logP" (ClogP) is determined by the fragment approach of Hansch and Leo (cf., A. Leo, in Comprehensive Medicinal Chemistry, Vol. 4, C. Hansch, P.G. Sammens, J.B. taylor, and C.A. Ramsden, Eds. P. 295, Pergamon Press, 1990, incorporated herein by reference). ClogP values may be calculated by using the "CLOGP" program available from Daylight Chemical Information Systems Inc. of Irvine, California U.S.A..

**(5) Boiling Point**

Boiling point is measured by ASTM method D2887-04a, "Standard Test Method for Boiling Range Distribution of Petroleum Fractions by Gas Chromatography," ASTM International.

**(6) Delivery Index Calculation**

The Delivery Index for a particle is calculated using the following equation:

$$Delivery\_Index = \frac{\left[\left(\frac{\mu}{\sigma}\right)_{Particle\_Size}\left(\frac{f_0}{f}\right)_{Fracture\_Stress}\left(\frac{L/L_0}{t/\mu}\right)\right]}{100}$$

Where

$\mu$ is the average particle diameter

$\sigma$ is the standard deviation of the average particle diameter

$f_0$ is the minimum in-use fracture strength required to break the microcapsule

$f$ is the measured Fracture Strength

$(L/L_0)/(t/\mu)$ is the Benefit Agent Retention Ratio

$t$ is the shell thickness of the particle

**(7) Odor Detection Threshold (ODT)**

Odour detection threshold is determined using the protocol found in U.S. Patent 6,869,923 B1, from Column 3, line 39 through Column 4, line 15.

EXAMPLES

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

| Material Name | Example 1 | Example 2 | Example 3 |
|---|---|---|---|
| | (class I) | (class I&II) | (class I&II) |
| 1,1-Dimethoxy-2,2,5-trimethyl-4-hexene (methyl pamplemouse) | 4 | 4 | 5 |
| Geraniol | 18 | 15 | 10 |
| (-)-Menthol | 0.1 | 0.1 | 0.1 |
| 4-Methyl-3-decen-5-ol(Undecavertol) | 3 | 3 | 3 |
| Dihydromyrcenol | 5 | 5 | 0 |
| Ionone gamma Methyl | 10 | 10 | 10 |
| Tetrahydrolinalool | 20 | 20 | 15 |
| 2,6,10-Trimethyl-9-undecenal (Adoxal) | | | 1 |
| Citral | | | 6 |
| alpha,alpha-Dimethyl-p-ethylphenylpropanal (Floralozone) | 2 | 2 | 3 |
| 2,4-Dimethyl-3-cyclohexene-1-carboxaldehyde (Ligustral) | 3 | 3 | 3 |
| Methyl n-nonyl acetaldehyde | 0.3 | 0.3 | 0.6 |
| Decyl Aldehyde | 0.3 | 0.3 | 0.6 |
| Intreleven Aldehyde | 0.3 | 0.3 | 0.6 |
| Lauric Aldehyde | 0.4 | 0.4 | 0.7 |
| Allyl cyclohexanepropionate | 2 | 2 | 3 |
| 2-tert.Butylcyclohexyl acetate | 20 | 10 | 3 |

(continued)

| Material Name | Example 1 (class I) | Example 2 (class I&II) | Example 3 (class I&II) |
|---|---|---|---|
| Tricyclodecenyl propionate / Frutene | 6 | 6 | 10 |
| Eucalyptol | 2 | 2 | 3 |
| Rose oxide | 0.1 | 0.1 | 0.5 |
| Isomenthone | | | 0.1 |
| gamma-Undecalactone | 2 | 2 | 3 |
| Citronellyl nitrile | 0.5 | 0.5 | 0.5 |
| 5-Methyl-3-heptanone oxime (stemone) | | | 0.3 |
| Dihydroeugenol | 1 | 1 | 1 |
| Class II | | | |
| Ethyl-2-methyl butyrate | | 1 | 2 |
| Cinnamic alcohol | | 1 | 1 |
| Hexenol; 3-Hexen-1-ol; cis-3-Hexenol;(Z)-3-Hexenol; | | 1 | 1 |
| ethyl vanillin | | 2 | 3 |
| Methyl anthranilate | | 1 | 1 |
| Maltol | | 1 | 2 |
| Allyl cyclohexyloxyacetate (cyclo galbanate) | | 4 | 3 |
| coumarin | | 2 | 4 |
| Total | 100 | 100 | 100 |

EXAMPLE 4: Urea Based Polyurea Capsule (outside the scope of the claims)

2 grams of Urea (Sigma Aldrich of Milwaukee, WI) is dissolved in 20g deionized water. 1 gram of resorcinol (Sigma Aldrich of Milwaukee, WI) is added to the homogeneous urea solution. 20 g of 37wt% formaldehyde solution (Sigma Aldrich of Milwaukee, WI) is added to the solution, and the pH of the slurry is adjusted to 8.0 using 1M sodium hydroxide solution (Sigma Aldrich of Milwaukee, WI). The reactants are allowed to sit at 35°C for 2 hours. In a separate beaker, 80 grams of fragrance oil of Example 1 is added slowly to the urea-formaldehyde solution. The mixture is agitated using a Janke & Kunkel Laboretechnik mixer using a pitched, 3-blade agitator to achieve a 50 micron mean oil droplet size distribution. The pH of the slurry is adjusted to 3.0 using 1M Hydrochloric Acid to initiate the condensation reaction. The solution is heated to 65°C and allowed to react in a constant temperature water bath, while slowly agitating the contents of the mixture. The contents are allowed to react for 4 hours at 65°C.

EXAMPLE 5: Melamine based Polyurea capsule (85% core / 15% wall)

A first mixture is prepared by combining 208 grams of water and 5 grams of alkyl acrylate-acrylic acid copolymer ( Polysciences, Inc. of Warrington, Pennsylvania, USA). This first mixture is adjusted to pH 5.0 using acetic acid.
178 grams of the capsule core material which comprise a fragrance oil of Example 1 is added to the first mixture at a temperature of 45°C to form an emulsion. The ingredients to form the capsule wall material are prepared as follows: 9 grams of a corresponding capsule wall material copolymer pre-polymer (butylacrylate-acrylic acid copolymer) and 90 grams of water are combined and adjusted to pH 5.0. To this mixture is added 28 grams of a partially methylated methylol melamine resin solution ("Cymel 385", 80% solids, Cytec). This mixture is added to the above described fragrance oil-in-water emulsion with stirring at a temperature of 45 degrees Centigrade. High speed blending is used to achieve a volume-mean particle size of 15 micron, and a standard deviation of 2 microns. The temperature of the mixture is gradually raised to 65 degrees Centigrade, and is maintained at this temperature for 8 hours with continuous stirring to initiate and complete encapsulation.
To form the acrylic acid-alkyl acrylate copolymer capsule wall, the alkyl group can be selected from ethyl, propyl, butyl, amyl, hexyl, cyclohexyl, 2-ethylhexyl, or other alkyl groups having from one to about sixteen carbons, preferably one to eight carbons.

EXAMPLE 6: Melamine based Polyurea capsule (90% core / 10% wall)

Perfume capsules are manufactured by the method of Example 5 with the exception that 280 grams of capsule core material of Example 1 is added to the first mixture. The resulting aqueous slurry of microcapsules have a volume-mean particle size of 14 micron, and a standard deviation of 2.6 microns.

EXAMPLE 7: Melamine-based Polyurea capsule (95% core/5% wall)

Perfume capsules are manufactured by the method of Example 5 with the exception that 280 grams of capsule core material of Example 1 is added to the first mixture. The resulting aqueous slurry of microcapsules have a volume-mean particle size of 11 micron, and a standard deviation of 3.2 microns.

EXAMPLE 8: Melamine based Polyurea capsule

Perfume capsules are manufactured by the method of Example 5 using fragrance composition of Example 2.

EXAMPLE 9: Melamine based Polyurea capsule

Perfume capsules are manufactured by the method of Example 5 using fragrance composition of Example 3.

EXAMPLE 10: Melamine based Polyurea capsule (85% core / 15% wall)

A first mixture is prepared by combining 208 grams of water and 5 grams of alkyl acrylate-acrylic acid copolymer ( Polysciences, Inc. of Warrington, Pennsylvania, USA). This first mixture is adjusted to pH 5.0 using sodium hydroxide.
178 grams of the capsule core material which comprise a fragrance oil of Example 1 is added to the first mixture at a temperature of 65°C to form an emulsion. High speed blending is used to achieve a volume-mean particle size of 15 microns. The ingredients to form the capsule wall material are prepared as follows: 9 grams of a corresponding capsule wall material copolymer pre-polymer (butylacrylate-acrylic acid copolymer) and 90 grams of water are combined and adjusted to pH 5.0. To this mixture is added 28 grams of a partially methylated methylol melamine resin solution ("Cymel 385", 80% solids, Cytec). This mixture is added to the above described fragrance oil-in-water emulsion with stirring at a temperature of 65 degrees Centigrade. The temperature of the mixture is maintained at this temperature for 8 hours with continuous stirring to initiate and complete encapsulation.
To form the acrylic acid-alkyl acrylate copolymer capsule wall, the alkyl group can be selected from ethyl, propyl, butyl, amyl, hexyl, cyclohexyl, 2-ethylhexyl, or other alkyl groups having from one to about sixteen carbons, preferably one to eight carbons.

EXAMPLE 11: Melamine based Polyurea capsule (90% core / 10% wall)

Perfume capsules are manufactured by the method of Example 10 with the exception that 280 grams of capsule core material of Example 1 is added to the first mixture. The resulting aqueous slurry of microcapsules have a volume-mean particle size of 14 micron, and a standard deviation of 2.6 microns.

EXAMPLE 12: Melamine-based Polyurea capsule (95% core/5% wall)

Perfume capsules are manufactured by the method of Example 10 with the exception that 280 grams of capsule core material of Example 1 is added to the first mixture. The resulting aqueous slurry of microcapsules have a volume-mean particle size of 11 micron, and a standard deviation of 3.2 microns.

EXAMPLE 13 Applying a coating of sodium silicate onto a microcapsule

To 171 grams of a dispersion of microcapsules containing 47wt% microcapsule particles of Example 5 is added 45 grams of sodium silicate 3.2R solution (44wt% active, obtained from Aldrich, P.O. Box 2060, Milwaukee, WI 53201, USA.) 154 grams of Deionized water is added to the slurry, and then pumped through a peristaltic pump into a centrifugal wheel nozzle rotating at 25,000 RPM, and situated in a co-current spray drying chamber (Niro, 3ft diameter). The atomized aqueous dispersion of microcapsules is spray dried at the following operating conditions: an inlet air temperature of 200°C, an outlet air temperature of 95°C, pressure drop of air is 42 millimeters of water (corresponds to 78 kg/hr airflow), the spray dryer is operated under a net negative pressure of-150 millimeters of water, and the pressure of air fed to the centrifugal atomizer is 5.0 barg. The dry particles are recovered from the collection vessel at the bottom of the spray dryer as well as from the cyclone, and mixed to form a homogeneous powder sample. The particles are found to have an average particle diameter of 50 micrometers. When the powder is added to a fabric care composition and aged for 4 weeks at 40°C, less than 10% perfume loss is observed from the microcapsule particles.

EXAMPLE 14 Fabric Conditioning Compositions

The following are non-limiting examples of the fabric conditioning compositions of the present invention.

| (%wt) | I | II | III | IV | V | VI | VIII |
|---|---|---|---|---|---|---|---|
| FSA [a] | 14-16.5 | 14-16.5 | 14-16.5 | 14-16.5 | 14-16.5 | 14-16.5 | 14-16.5 |
| Ethanol | 2.2-2.6 | 2.2-2.6 | 2.2-2.6 | 2.2-2.6 | 2.2-2.6 | 2.2-2.6 | 2.2-2.6 |
| Starch [b] | 1.25-1.5 | 1.25-1.5 | 1.25-1.5 | 1.25-1.5 | 1.25-1.5 | 1.25-1.5 | 1.25-1.5 |
| Perfume | 0.8-1.5 | 0.8-1.5 | 0.8-1.5 | 0.8-1.5 | 0.8-1.5 | 0.8-1.5 | 0.8-1.5 |
| Encapsulated Perfume | 0.6 | 0.6 | 0.6 | 0.6 | 0.6 | 0.60 | 0.6 |
| Phase Stabilizing Polymer [c] | 0.14-0.21 | 0.14-0.21 | 0.14-0.21 | 0.14-0.21 | 0.14-0.21 | 0.14-0.21 | 0.14-0.21 |
| Calcium Chloride | 0.1-0.3 | 0.1-0.3 | 0.1-0.3 | 0.1-0.3 | 0.1-0.3 | 0.1-0.3 | 0.1-0.3 |
| DTPA [d] | 0.017 | 0.017 | 0.017 | 0.017 | 0.017 | 0.017 | 0.017 |
| Preservative (ppm) [e] | 5 | 5 | 5 | 5 | 5 | 5 | 5 |
| Antifoam [f] | 0.015 | 0.018 | 0.015 | 0.015 | 0.015 | 0.015 | 0.015 |
| Dye (ppm) | 30-300 | 30-300 | 30-300 | 30-300 | 30-300 | 30-300 | 30-300 |
| Ammonium Chloride | 0.02-0.12 | 0.02-0.12 | 0.02-0.12 | 0.02-0.12 | 0.02-0.12 | 0.02-0.12 | 0.02-0.12 |
| HCl | 0.012 | 0.014 | 0.012 | 0.012 | 0.028 | 0.028 | 0.025 |
| Structurant [g] | 0.01 | 0.01 | 0.01 | 0.01 | 0.01 | 0.01 | 0.01 |
| Deionized Water | Balance | Balance | Balance | Balance | Balance | Balance | Balance |
| Microcapsule Example # | 4 | 5 | 6 | 7 | 8 | 9 | 10 |

[a] N,N-di(tallowoyloxyethyl)-N,N-dimethylammonium chloride.
[b] Cationic high amylose maize starch available from National Starch under the trade name CATO®.
[c] Copolymer of ethylene oxide and terephthalate having the formula described in US 5,574,179 at col.15, lines 1-5, wherein each X is methyl, each n is 40, u is 4, each R1 is essentially 1,4-phenylene moieties, each R2 is essentially ethylene, 1,2-propylene moieties, or mixtures thereof. [d] Diethylenetriaminepentaacetic acid.
[c] KATHON® CG available from Rohm and Haas Co. "PPM" is "parts per million."
[f] Silicone antifoam agent available from Dow Coming Corp. under the trade name DC2310.
[g] Hydrophobically-modified ethoxylated urethane available from Rohm and Haas under the tradename Aculan 44.

EXAMPLE 15: Fabric Conditioner Compositions

Non-limiting examples of product formulations containing microcapsules are summarized in the following table.

| (%wt) | XIV | XV | XVI | XVII | XVIII | XIX | XX |
|---|---|---|---|---|---|---|---|
| FSA [a] | 12 | 12 | 16.47 | --- | --- | 5 | 5 |
| FSA [b] | | --- | | 3.00 | --- | --- | --- |
| FSA [c] | | --- | | --- | 6.5 | --- | --- |

(continued)

| (%wt) | XIV | XV | XVI | XVII | XVIII | XIX | XX |
|---|---|---|---|---|---|---|---|
| Ethanol | 1.95 | 1.95 | 2.57 | --- | --- | 0.81 | 0.81 |
| Isopropyl Alcohol | --- | --- | --- | 0.33 | 1.22 | --- | --- |
| Starch <sup>d</sup> | 1.25 | --- | 2.30 | 0.5 | 0.70 | 0.71 | 0.42 |
| Encapsulated Perfume of Example 5 | 0.75 0.37 | | | 0.60 | 0.37 | 0.6 | 0.37 | 0.37 |
| Formaldehyde Scavenger<sup>e</sup> | 0.25 | 0.03 | 0.030 | 0.030 | 0.065 | 0.03 | 0.03 |
| Phase Stabilizing Polymer <sup>f</sup> | 0.21 | 0.14 | --- | --- | 0.14 | --- | --- |
| Suds Suppressor <sup>g</sup> | --- | --- | --- | --- | 0.1 | --- | --- |
| Calcium Chloride | 0.15 | 0.30 | 0.176 | --- | 0.1-0.15 | --- | --- |
| DTPA <sup>h</sup> | 0.017 | 0.007 | 0.007 | 0.20 | --- | 0.002 | 0.002 |
| Preservative (ppm) <sup>i,j</sup> | 5 | 5 | 5 | --- | 250 <sup>j</sup> | 5 | 5 |
| Antifoam<sup>k</sup> | 0.015 | 0.015 | 0.015 | --- | --- | 0.015 | 0.015 |
| Dye (ppm) | 40 | 40 | 40 | 11 | 30-300 | 30 | 30 |
| Ammonium Chloride | 0.100 | 0.115 | 0.115 | --- | --- | --- | --- |
| HCl | 0.012 | 0.028 | 0.028 | 0.016 | 0.025 | 0.011 | 0.011 |
| Structurant<sup>l</sup> | 0.01 | 0.01 | 0.01 | 0.01 | 0.01 | 0.01 | 0.01 |
| Deionized Water | Balance | Balance | Balance | Balance | Balance | Balance | Balance |

<sup>a</sup> N,N-di(tallowoyloxyethyl)-N,N-dimethylammonium chloride.

<sup>b</sup> Methyl bis(tallow amidoethyl)2-hydroxyethyl ammonium methyl sulfate.

<sup>c</sup> Reaction product of Fatty acid with Methyldiethanolamine in a molar ratio 1.5:1, quaternized with Methylchloride, resulting in a 1:1 molar mixture of N,N-bis(stearoyl-oxy-ethyl) N,N-dimethyl ammonium chloride and N-(stearoyl-oxy-ethyl) N,-hydroxyethyl N,N dimethyl ammonium chloride.

<sup>d</sup> Cationic high amylose maize starch available from National Starch under the trade name CATO®.

<sup>e</sup> The formaldehyde scavenger is acetoacetamide available from Aldrich.

<sup>f</sup> Copolymer of ethylene oxide and terephthalate having the formula described in US 5,574,179 at col.15, lines 1-5, wherein each X is methyl, each n is 40, u is 4, each R1 is essentially 1,4-phenylene moieties, each R2 is essentially ethylene, 1,2-propylene moieties, or mixtures thereof. <sup>g</sup> SE39 from Wacker

<sup>h</sup> Diethylenetriaminepentaacetic acid.

<sup>i</sup> KATHON® CG available from Rohm and Haas Co. "PPM" is "parts per million."

<sup>j</sup> Gluteraldehyde

<sup>k</sup> Silicone antifoam agent available from Dow Coming Corp. under the trade name DC2310.

<sup>l</sup> Hydrophobically-modified ethoxylated urethane available from Rohm and Haas under the tradename Aculan 44.

In the detergent compositions, the abbreviated component identifications have the following meanings:

| | |
|---|---|
| LAS : | Sodium linear C11-13 alkyl benzene sulfonate |
| TAS : | Sodium tallow alkyl sulfate |
| CxyAS : | Sodium $C_{1x}$ - $C_{1y}$ alkyl sulfate |
| C46SAS : | Sodium C14 - C16 secondary (2,3) alkyl sulfate |
| CxyEzS : | Sodium C1x-C1y alkyl sulfate condensed with z moles of ethylene oxide |
| CxyEz : | C1x-C1y predominantly linear primary alcohol condensed with an average of z moles of ethylene oxide |
| QAS : | R2.N+(CH3)2(C2H4OH) with R2 = C12 - C14 |
| QAS 1 : | R2.N+(CH3)2(C2H4OH) with R2 = C8 - C 11 |
| APA : | C8 - C 10 amido propyl dimethyl amine |
| Soap : | Sodium linear alkyl carboxylate derived from an 80/20 mixture of tallow and coconut fatty acids |

| | |
|---|---|
| STS : | Sodium toluene sulphonate |
| CFAA : | C12-C14 (coco) alkyl N-methyl glucamide |
| TFAA : | C16-C18 alkyl N-methyl glucamide |
| TPKFA : | C12-C14 topped whole cut fatty acids |
| STPP : | Anhydrous sodium tripolyphosphate |
| TSPP : | Tetrasodium pyrophosphate |
| Zeolite A : | Hydrated sodium aluminosilicate of formula Nal2(AlO2SiO2)12.27H2O having a primary particle size in the range from 0.1 to 10 micrometers (weight expressed on an anhydrous basis) |
| NaSKS-6 : | Crystalline layered silicate of formula δ- Na2Si2O5 |
| Citric acid : | Anhydrous citric acid |
| Borate : | Sodium borate |
| Carbonate : | Anydrous sodium carbonate with a particle size between $200\mu m$ and $900\mu m$ |
| Bicarbonate : | Anhydrous sodium bicarbonate with a particle size distribution between $400\mu m$ and $1200\mu m$ |
| Silicate : | Amorphous sodium silicate (SiO2:Na2O = 2.0:1) |
| Sulfate : | Anhydrous sodium sulfate |
| Mg sulfate : | Anhydrous magnesium sulfate |
| Citrate : | Tri-sodium citrate dihydrate of activity 86.4% with a particle size distribution between $425\mu m$ and $850\mu m$ |
| MA/AA : | Copolymer of 1:4 maleic/acrylic acid, average molecular weight about 70,000 |
| MA/AA (1) : | Copolymer of 4:6 maleic/acrylic acid, average molecular weight about 10,000 |
| AA : | Sodium polyacrylate polymer of average molecular weight 4,500 |
| CMC : | Sodium carboxymethyl cellulose |
| Cellulose ether : | Methyl cellulose ether with a degree of polymerization of 650 available from Shin Etsu Chemicals |
| Protease : | Proteolytic enzyme, having 3.3% by weight of active enzyme, sold by NOVO Industries A/S under the tradename Savinase |
| Protease I : | Proteolytic enzyme, having 4% by weight of active enzyme, as described in WO 95/10591, sold by Genencor Int. Inc. |
| Alcalase : | Proteolytic enzyme, having 5.3% by weight of active enzyme, sold by NOVO Industries A/S |
| Cellulase : | Cellulytic enzyme, having 0.23% by weight of active enzyme, sold by NOVO Industries A/S under the tradename Carezyme |
| Amylase : | Amylolytic enzyme, having 1.6% by weight of active enzyme, sold by NOVO Industries A/S under the tradename Termamyl 120T |
| Lipase : | Lipolytic enzyme, having 2.0% by weight of active enzyme, sold by NOVO Industries A/S under the tradename Lipolase |
| Lipase (1) : | Lipolytic enzyme, having 2.0% by weight of active enzyme, sold by NOVO Industries A/S under the tradename Lipolase Ultra |
| Endolase : | Endoglucanase enzyme, having 1.5% by weight of active enzyme, sold by NOVO Industries A/S |
| PB4 : | Sodium perborate tetrahydrate of nominal formula NaBO2.3H2O.H2O2 |
| PB1 : | Anhydrous sodium perborate bleach of nominal formula NaBO2.H2O2 |
| Percarbonate : | Sodium percarbonate of nominal formula 2Na2CO3.3H2O2 |
| NOBS : | Nonanoyloxybenzene sulfonate in the form of the sodium salt |
| NAC-OBS : | (6-nonamidocaproyl) oxybenzene sulfonate |
| TAED : | Tetraacetylethylenediamine |
| DTPA : | Diethylene triamine pentaacetic acid |
| DTPMP : | Diethylene triamine penta (methylene phosphonate), marketed by Monsanto under the Tradename Dequest 2060 |
| EDDS : | Ethylenediamine-N,N'-disuccinic acid, (S,S) isomer in the form of its sodium salt. |
| Photoactivated : bleach (1) | Sulfonated zinc phthlocyanine encapsulated in dextrin soluble polymer |
| Photoactivated : bleach (2) | Sulfonated alumino phthlocyanine encapsulated in dextrin soluble polymer |
| Brightener 1 : | Disodium 4,4'-bis(2-sulphostyryl)biphenyl |
| Brightener 2 : | Disodium 4,4'-bis(4-anilino-6-morpholino-1.3.5-triazin-2- yl)amino) stilbene-2:2'-disulfonate |
| HEDP : | 1,1-hydroxyethane diphosphonic acid |
| PEGx : | Polyethylene glycol, with a molecular weight of x (typically 4,000) |

PEO :        Polyethylene oxide, with an average molecular weight of 50,000
TEPAE :       Tetraethylenepentaamine ethoxylate
PVI :         Polyvinyl imidosole, with an average molecular weight of 20,000
PVP :        Polyvinylpyrolidone polymer, with an average molecular weight of 60,000
PVNO :       Polyvinylpyridine N-oxide polymer, with an average molecular weight of 50,000
PVPVI :       Copolymer of polyvinylpyrolidone and vinylimidazole, with an average molecular weight of 20,000
QEA :         bis((C2H5O)(C2H4O)n)(CH3) -N+-C6H12-N+-(CH3) bis((C2H5O)-(C2H4O))n, wherein n = from 20 to 30
SRP 1 :       Anionically end capped poly esters
SRP 2 :       Diethoxylated poly (1, 2 propylene terephtalate) short block polymer
PEI :         Polyethyleneimine with an average molecular weight of 1800 and an average ethoxylation degree of 7 ethyleneoxy residues per nitrogen
Silicone antifoam :       Polydimethylsiloxane foam controller with siloxane-oxyalkylene copolymer as dispersing agent with a ratio of said foam controller to said dispersing agent of 10:1 to 100:1
Opacifier :       Water based monostyrene latex mixture, sold by BASF Aktiengesellschaft under the tradename Lytron 621
Wax :         Paraffin wax

| DEQA | Di-(tallow-oxy-ethyl) dimethyl ammonium chloride. |
|---|---|
| DEQA (2) | Di-(soft-tallowyloxyethyl) hydroxyethyl methyl ammonium methylsulfate. |
| DTDMAMS | Ditallow dimethyl ammonium methylsulfate. |
| SDASA | 1:2 ratio of stearyldimethyl amine:triple-pressed stearic acid. |
| PA30 | Polyacrylic acid of average molecular weight of between about 4,500 - 8,000. |
| 480N | Random copolymer of 7:3 acrylate/methacrylate, average molecular weight about 3,500. |
| Polygel/carbopol | High molecular weight crosslinked polyacrylates. |
| Metasilicate | Sodium metasilicate (SiO2:Na2O ratio = 1.0). |
| Nonionic | C13-C15 mixed ethoxylated/propoxylated fatty alcohol with an average degree of ethoxylation of 3.8 and an average degree of propoxylation of 4.5. |
| Neodol 45-13 | C14-C15 linear primary alcohol ethoxylate, sold by Shell Chemical CO. |
| MnTACN | Manganese 1,4,7-trimethyl-1,4,7-triazacyclononane. |
| PAAC | Pentaamine acetate cobalt(III) salt. |
| Paraffin | Paraffin oil sold under the tradename Winog 70 by Wintershall. |
| NaBz | Sodium benzoate. |
| BzP | Benzoyl Peroxide. |
| SCS | Sodium cumene sulphonate. |
| BTA | Benzotriazole. |
| pH | Measured as a 1% solution in distilled water at 20°C. |

EXAMPLE 16

The following high density granular laundry detergent compositions A to F are prepared in accord with the invention:

| | A | B | C | D |
|---|---|---|---|---|
| Blown powder | | | | |
| LAS | 6.0 | 5.0 | 11.0 | 6.0 |

(continued)

|  | A | B | C | D |
|---|---|---|---|---|
| Blown powder |  |  |  |  |
| TAS | 2.0 | - | - | 2.0 |
| Zeolite A | 24.0 | - | - | 20.0 |
| STPP | - | 27.0 | 24.0 | - |
| Sulfate | 4.0 | 6.0 | 13.0 | - |
| MA/AA | 1.0 | 4.0 | 6.0 | 2.0 |
| Silicate | 1.0 | 7.0 | 3.0 | 3.0 |
| CMC | 1.0 | 1.0 | 0.5 | 0.6 |
| Brightener 1 | 0.2 | 0.2 | 0.2 | 0.2 |
| Silicone antifoam | 1.0 | 1.0 | 1.0 | 0.3 |
| DTPMP | 0.4 | 0.4 | 0.2 | 0.4 |
| Spray on |  |  |  |  |
| Brightener | 0.02 | - | - | 0.02 |
| C45E7 | - | - | - | 5.0 |
| C45E2 | 2.5 | 2.5 | 2.0 | - |
| C45E3 | 2.6 | 2.5 | 2.0 | - |
| Perfume | 0.5 | 0.3 | 0.5 | 0.2 |
| Silicone antifoam | 0.3 | 0.3 | 0.3 | - |
| Dry additives |  |  |  |  |
| QEA | - | - | - | 1.0 |
| EDDS | 0.3 | - | - | - |
| Sulfate | 2.0 | 3.0 | 5.0 | 10.0 |
| Carbonate | 6.0 | 13.0 | 15.0 | 14.0 |
| Citric acid | 2.5 | - | - | 2.0 |
| QAS II | 0.5 | - | - | 0.5 |
| SKS-6 | 10.0 | - | - | - |
| Percarbonate | 18.5 | - | - | - |
| PB4 | - | 18.0 | 10.0 | 21.5 |
| TAED | 2.0 | 2.0 | - | 2.0 |
| NAC-OBS | 3.0 | 2.0 | 4.0 | - |
| Protease | 1.0 | 1.0 | 1.0 | 1.0 |
| Lipase | - | 0.4 | - | 0.2 |
| Lipase (1) | 0.4 | - | 0.4 | - |
| Amylase | 0.2 | 0.2 | 0.2 | 0.4 |
| Brightener 1 | 0.05 | - | - | 0.05 |
| Encapsulated Perfume of Example 5 | 0.1 | 0.3 | 0.15 | 0.4 |

(continued)

| Dry additives | | | | |
|---|---|---|---|---|
| Misc/minor to 100% | | | | |

EXAMPLE 17 Heavy Duty Liquid Detergent Composition

The following liquid detergent formulations are prepared in accord with the invention (levels are given as parts per weight).

| | G | H | I | J | K |
|---|---|---|---|---|---|
| LAS | 11.5 | 8.8 | - | 3.9 | - |
| C25E2.5S | - | 3.0 | 18.0 | - | 16.0 |
| C45E2.25S | 11.5 | 3.0 | - | 15.7 | - |
| C23E9 | - | 2.7 | 1.8 | 2.0 | 1.0 |
| C23E7 | 3.2 | - | - | - | - |
| CFAA | - | - | 5.2 | - | 3.1 |
| TPKFA | 1.6 | - | 2.0 | 0.5 | 2.0 |
| Citric acid (50%) | 6.5 | 1.2 | 2.5 | 4.4 | 2.5 |
| Calcium formate | 0.1 | 0.06 | 0.1 | - | - |
| Sodium formate | 0.5 | 0.06 | 0.1 | 0.05 | 0.05 |
| Sodium cumene sulfonate | 4.0 | 1.0 | 3.0 | 1.18 | - |
| Borate | 0.6 | - | 3.0 | 2.0 | 2.9 |
| Sodium hydroxide | 5.8 | 2.0 | 3.5 | 3.7 | 2.7 |
| Ethanol | 1.75 | 1.0 | 3.6 | 4.2 | 2.9 |
| 1, 2 propanediol | 3.3 | 2.0 | 8.0 | 7.9 | 5.3 |
| Monoethanolamine | 3.0 | 1.5 | 1.3 | 2.5 | 0.8 |
| TEPAE | 1.6 | - | 1.3 | 1.2 | 1.2 |
| Protease | 1.0 | 0.3 | 1.0 | 0.5 | 0.7 |
| Lipase | - | - | 0.1 | - | - |
| Cellulase | - | - | 0.1 | 0.2 | 0.05 |
| Amylase | - | - | - | 0.1 | - |
| SRP 1 | 0.2 | - | 0.1 | - | - |
| DTPA | - | - | 0.3 | - | - |
| PVNO | - | - | 0.3 | - | 0.2 |
| Perfume | 0.4 | 0.4 | 0.4 | 0.4 | 0.4 |
| Encapsulated Perfume of Example 5 | 0.2 | 0.5 | 0.1 | 0.3 | 0.1 |
| Brightener 1 | 0.2 | 0.07 | 0.1 | - | - |
| Silicone antifoam | 0.04 | 0.02 | 0.1 | 0.1 | 0.1 |
| Water/minors | | | | | |

EXAMPLE 18 Substrate based composition

The following fabric softener and dryer added fabric conditioner compositions are prepared according to the present invention:

| | L | M | N | O | P |
|---|---|---|---|---|---|
| DEQA | 2.6 | 19.0 | - | - | - |
| DEQA(2) | - | - | - | - | 51.8 |
| DTMAMS | - | - | - | 26.0 | - |
| SDASA | - | - | 70.0 | 42.0 | 40.2 |
| Stearic acid of IV=0 | 0.3 | - | - | - | - |
| Neodol 45-13 | - | - | 13.0 | - | - |
| Hydrochloride acid | 0.02 | 0.02 | - | - | - |
| Ethanol | - | - | 1.0 | - | - |
| Encapsulated Perfume of Example 5 | 0.2 | 0.4 | 0.6 | 0.2 | 0.2 |
| Perfume | 1.0 | 1.0 | 0.75 | 1.0 | 1.5 |
| Glycoperse S-20 | - | - | - | - | 15.4 |
| Glycerol monostearate | - | - | - | 26.0 | - |
| Digeranyl Succinate | - | - | 0.38 | - | - |
| Silicone antifoam | 0.01 | 0.01 | - | - | - |
| Electrolyte | - | 0.1 | - | - | - |
| Clay | - | - | - | 3.0 | - |
| Dye | 10ppm | 25ppm | 0.01 | - | - |
| Water and minors | 100% | 100% | - | - | - |

EXAMPLE 19 Hard Surface Cleaning Compositions

The following liquid hard surface cleaning compositions are prepared according to the present invention:

| | Q | R | S | T | U |
|---|---|---|---|---|---|
| Encapsulated Perfume of Example 5 | 0.3 | 0.3 | 0.5 | 0.3 | 0.3 |
| Amylase | 0.01 | 0.002 | 0.005 | - | - |
| Protease | 0.05 | 0.01 | 0.02 | - | - |
| Hydrogen peroxide | - | - | - | 6.0 | 6.8 |
| Acetyl triethyl citrate | - | - | - | 2.5 | - |
| DTPA | - | - | - | 0.2 | - |
| Butyl hydroxy toluene | - | - | - | 0.05 | - |
| EDTA* | 0.05 | 0.05 | 0.05 | - | - |
| Citric / Citrate | 2.9 | 2.9 | 2.9 | 1.0 | - |
| LAS | 0.5 | 0.5 | 0.5 | - | - |
| C12 AS | 0.5 | 0.5 | 0.5 | - | - |
| C10AS | - | - | - | - | 1.7 |
| C12(E)S | 0.5 | 0.5 | 0.5 | - | - |

(continued)

| | Q | R | S | T | U |
|---|---|---|---|---|---|
| C12,13 E6.5 nonionic | 7.0 | 7.0 | 7.0 | - | - |
| Neodol 23-6.5 | - | - | - | 12.0 | - |
| Dobanol 23-3 | - | - | - | - | 1.5 |
| Dobanol 91-10 | - | - | - | - | 1.6 |
| C25AE1.8S | - | - | - | 6.0 | |
| Na paraffin sulphonate | - | - | - | 6.0 | |
| Perfume | - | 1.0 | 1.0 | 0.5 | 0.2 |
| Propanediol | - | - | - | 1.5 | |
| Ethoxylated tetraethylene pentaimine | - | - | - | 1.0 | - |
| 2, Butyl octanol | - | - | - | - | 0.5 |
| Hexyl carbitol** | 1.0 | 1.0 | 1.0 | - | - |
| SCS | 1.3 | 1.3 | 1.3 | - | - |
| pH adjusted to | 7-12 | 7-12 | 7-12 | 4 | - |
| Miscellaneous and water Up to 100%<br>*Na4 ethylenediamine diacetic acid<br>**Diethylene glycol monohexyl ether | | | | | |

EXAMPLE 20

The following spray composition for cleaning of hard surfaces and removing household mildew is prepared according to the present invention:

| Material | Composition (wt%) |
|---|---|
| Encapsulated Perfume of Example 5 | 0.1 |
| Amylase | 0.01 |
| Protease | 0.01 |
| Na octyl sulfate | 2.0 |
| Na dodecyl sulfate | 4.0 |
| Na hydroxide | 0.8 |
| Silicate | 0.04 |
| Butyl carbitol* | 4.0 |
| Perfume | 0.35 |
| Water/minors | up to 100% |

EXAMPLE 21

The following toilet bowl cleaning composition is prepared according to the present invention.

| | CN | CO |
|---|---|---|
| C14-15 linear alcohol 7EO | 2.0 | 10.0 |
| Citric acid | 10.0 | 5.0 |

(continued)

|  | CN | CO |
|---|---|---|
| HIA I | 1.0 | 2.0 |
| DTPMP | - | 1.0 |
| Dye | 2.0 | 1.0 |
| Perfume | 3.0 | 3.0 |
| NaOH | pH 6-11 | |
| Water and minors | Up to 100% | |

EXAMPLE 22

Shampoos and conditioners of the present invention may be made in accordance with the teachings of US 2003/0108501 A1.

For example, the shampoos of the present invention may be made in accordance with the making instructions on pages 19 and 20. For example, Example 9 of US 2003/0108501 A1 may be used except the particle "Expancel at 1%" is replaced with the perfume microcapsules of this invention at a level that gives 0.8% perfume in the shampoo.

EXAMPLE 23: Rinse Off Conditioner

|  | Ingredient |  | Wt. % |
|---|---|---|---|
| 1 | Perfume Microcapsules of the Present Invention |  | 1. |
| 2 | Hydroxyethyl stearate |  | 0.25 |
| 3 | Polyox WAR N-10 (PEG-2M) |  | 0.5 |
| 4 | Quaternium 18 |  | 0.75 |
| 5 | Stearamidopropyl dimethyl amine |  | 1 |
| 6 | Glyceryl monostearate |  | 0.25 |
| 7 | Polawax NF |  | 0.5 |
| 8 | Cetyl Alcohol |  | 0.96 |
| 9 | Stearyl Alcohol |  | 0.64 |
| 10 | Oleyl Alcohol |  | 0.25 |
| 11 | Acid EDTA |  | 0.1 |
| 12 | Benzyl Alcohol |  | 0.4 |
| 13 | Kathon CG |  | 0.033 |
| 14 | Silicone |  | 4.2 |
| 15 | Citric Acid |  | 0.13 |
| 16 | Water |  | Balance |

Conditioner making procedure:

Heat water to about 85°C.
Add ingredients 2 - 11 and mix 3 minutes at about 85°C.
Add ingredients 12 & 13 and mix 5 more minutes at about 85°C

Cool to 55°C.
Mix in ingredients 14 - 16.
Cool to room temperature.

EXAMPLE 24: Spray Hair Gel

|  | Ingredient | Wt. % |
|---|---|---|
| 1 | Perfume Microcapsules | 1 |
| 2 | Polyvinyl pyriolidone (PVP) | 2 |
| 3 | Lactic acid | 0.73 |
| 4 | Chitosan | 0.7 |
| 5 | DMDM Hydantoin | 0.5 |
| 6 | Peg-40 Hydrogenated Castor Oil | 0.4 |
| 7 | Polysorbate 20 | 0.4 |
| 8 | PEG-60 Almond Glycerides | 0.2 |
| 9 | Polysorbate 86 | 0.2 |
| 10 | Water | Balance |

A premix is made of the Chitosan, lactic acid & 15% of the batch's water.
A second premix is made of ingredients 6, 7, 8 & 10 at 40°C.
A main mix of water, Polysorbate 80, DMDM Hydantoin & PVP is prepared and then the Chitosan premix and Perfume microcapsule premixes are added to it.

EXAMPLE 25 Microcapsule Formation

Into 153 grams of a mixture of 149.5 grams of water and 3.5 grams of the acrylic acid-alkyl acrylate copolymer, adjusted to pH 5.0, are emulsified 180 grams of the intended capsule nucleus material solution of Table 2. A second mixture of 6.5 grams of the corresponding acrylic acid-alkyl acrylate copolymer and 65 grams of water is prepared and adjusted to pH 5.0 and 20 grams of a partially methylated methylol melamine resin solution ("Resimene 714", 80 percent solids, Monsanto Company, St. Louis, Mo.) is added and this mixture is in turn added with stirring to the above-described emulsion. The resulting mixture is placed in a container which is mounted in a room temperature water bath. Continuous stirring is provided and the bath is heated to 55 degrees C and maintained at this temperature, with continuous stirring, overnight to initiate and complete encapsulation. The resulting capsules are employed in any of the compositions of the present specification.

The dimensions and values disclosed herein are not to be understood as being strictly limited to the exact numerical values recited. Instead, unless otherwise specified, each such dimension is intended to mean both the recited value and functionally equivalent range surrounding that value. For example, a dimension disclosed as "40 mm" is intended to mean "about 40 mm".

The citation of any document is not to be construed as an admission that it is prior art with respect to the present invention. To the extent that any weaning or definition of the same term in a document, the meaning or definition assigned to that term in this document shall govern.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

**Claims**

1.   A particle comprising a benefit agent composition and a wall material comprising melamine cross-linked with formaldehyde that surrounds said benefit agent composition, said benefit agent composition comprising:

a.) from 50% of a first material having:

(i) a ClogP of from 2.5 to 6;
(ii) a boiling point of from 80 °C to less than 250 °C.

2. The particle of claim 1, further comprising a second material having:

(i) a ClogP of less than 2.5, of even less than 2 to 0.1; and
(ii) a ODT of less than 100 ppb, from 0.00001 ppb to less than 100 ppb, from 0.00001 ppb to less then 50 ppb or even from 0.00001 ppb to less than 20 ppb.

3. The particle of claim 2, wherein said second material has a boiling point of from 80 °C to 350 °C from 80 °C to 310 °C, from 80 °C to 230 °C, or even from 80 °C to 150 °C.

4. A particle of according to any preceding claim wherein said benefit agent composition comprises, based on total benefit agent composition weight, from 70%, 80%, 90% or even 100% of said first material.

5. A particle of claim 2 wherein any balance of said benefit agent composition comprises said second material.

6. A particle of claim 2 wherein said balance of said benefit agent composition comprises, based on total balance weight, 10%, 20%, 30%, 40%, 50%, 60%, 70%, 80%, 90% or even 100% of said second material.

7. A particle of according to any preceding claim, said particle having a Delivery Index of at least 0.05 at least 7, at least 70, or even from 500 to 2200.

8. A product and/or a composition comprising a particle according to any preceding claim and an adjunct material and/or a fabric softener active.

9. A composition comprising a particle according to any of claims 1-7, said composition being a consumer product.

10. A method of treating and/or cleaning a situs, said method comprising

a.) optionally washing and/or rinsing said situs;
b.) contacting said situs with a particle and/or composition according to any preceding claim; and
c.) optionally washing and/or rinsing said situs.

**Patentansprüche**

1. Teilchen, umfassend eine Wirkstoffzusammensetzung und ein Wandmaterial, das mit Formaldehyd vernetztes Melamin umfasst und die Wirkstoffzusammensetzung umgibt, wobei die Wirkstoffzusammensetzung Folgendes umfasst:

a.) zu 50 % ein erstes Material, das Folgendes aufweist:

(I) einen ClogP-Wert von 2,5 bis 6,
(II) einen Siedepunkt von 80 °C bis weniger als 250 °C.

2. Teilchen nach Anspruch 1, ferner umfassend ein zweites Material, das Folgendes aufweist:

(I) einen ClogP-Wert von weniger als 2,5 oder sogar weniger als 2 bis 0,1 und
(II) einen Geruchserkennungsschwellenwert von weniger als 100 ppb, von 0,00001 ppb bis weniger als 100 ppb, von 0,00001 ppb bis weniger als 50 ppb oder sogar von 0,00001 ppb bis weniger als 20 ppb.

3. Teilchen nach Anspruch 2, wobei das zweite Material einen Siedepunkt von 80 °C bis 350 °C, von 80 °C bis 310 °C, von 80 °C bis 230 °C oder sogar von 80 °C bis 150 °C aufweist.

4. Teilchen nach einem der vorstehenden Ansprüche, wobei die Wirkstoffzusammensetzung, basierend auf dem Gesamtgewicht der Wirkstoffzusammensetzung, zu 70 %, 80 %, 90 % oder sogar 100 % das erste Material umfasst.

5. Teilchen nach Anspruch 2, wobei ein Rest der Wirkstoffzusammensetzung das zweite Material umfasst.

6. Teilchen nach Anspruch 2, wobei der Rest der Wirkstoffzusammensetzung, basierend auf dem Gesamtgewicht des Rests, zu 10 %, 20 %, 30 %, 40 %, 50 %, 60 %, 70 %, 80 %, 90 % oder sogar 100 % das zweite Material umfasst.

7. Teilchen nach einem der vorstehenden Ansprüche, wobei das Teilchen einen Abgabeindex von mindestens 0,05, mindestens 7, mindestens 70 oder sogar von 500 bis 2200 aufweist.

8. Produkt und/oder Zusammensetzung, umfassend ein Teilchen nach einem der vorstehenden Ansprüche und ein Zusatzmaterial und/oder einen Gewebeweichmacher.

9. Zusammensetzung, umfassend ein Teilchen nach einem der Ansprüche 1 bis 7, wobei die Zusammensetzung ein Konsumprodukt ist.

10. Verfahren zum Behandeln und/oder Reinigen einer Stelle, wobei das Verfahren Folgendes umfasst:

   a.) wahlweise Waschen und/oder Spülen der Stelle,
   b.) Inkontaktbringen der Stelle mit einem Teilchen und/oder einer Zusammensetzung nach einem der vorstehenden Ansprüche und
   c.) wahlweise Waschen und/oder Spülen der Stelle.

**Revendications**

1. Particule comprenant une composition d'agent bénéfique et un matériau de paroi comprenant de la mélamine réticulée avec du formaldéhyde qui entoure ladite composition d'agent bénéfique, ladite composition d'agent bénéfique comprenant :

   a.) à partir de 50 % d'un premier matériau ayant :

      (i) un ClogP allant de 2,5 à 6 ;
      (ii) un point d'ébullition allant de 80 °C à moins de 250 °C.

2. Particule selon la revendication 1, comprenant, en outre, un deuxième matériau ayant :

   (i) un ClogP de moins de 2,5, ou même moins de 2 à 0,1 : et
   (ii) un SDO de moins de 100 parties par milliard, de 0,00001 partie par milliard à moins de 100 parties par milliard, de 0,00001 partie par milliard à moins de 50 parties par milliard ou même de 0,00001 partie par milliard à moins de 20 parties par milliard.

3. Particule selon la revendication 2, dans laquelle ledit deuxième matériau a un point d'ébullition allant de 80 °C à 350 °C, de 80 °C à 310 °C, de 80 °C à 230 °C, ou même de 80 °C à 150 °C.

4. Particule selon l'une quelconque des revendications précédentes, dans laquelle ladite composition d'agent bénéfique comprend, sur base du poids total de la composition d'agent bénéfique, à partir de 70 %, 80 %, 90 % ou même 100 % dudit premier matériau.

5. Particule selon la revendication 2, dans laquelle n'importe quel solde de ladite composition d'agent bénéfique comprend ledit deuxième matériau.

6. Particule selon la revendication 2, dans laquelle ledit solde de ladite composition d'agent bénéfique comprend, sur base du poids total du solde, 10 %, 20 %, 30 %, 40 %, 50 %, 60 %, 70 %, 80 %, 90 % ou même 100 % dudit deuxième matériau.

7. Particule selon l'une quelconque des revendications précédentes, ladite particule ayant un indice de libération d'au moins 0,05 au moins 7, au moins 70, ou même de 500 à 2200.

8. Produit et/ou composition comprenant une particule selon l'une quelconque des revendications précédentes et un matériau additif et/ou un agent actif adoucissant des tissus.

9. Composition comprenant une particule selon l'une quelconque des revendications 1 à 7, ladite composition étant un produit de consommation.

10. Procédé de traitement et/ou de nettoyage d'un site, ledit procédé comprenant

a.) facultativement laver et/ou rincer ledit site ;
b.) mettre en contact ledit site avec une particule et/ou une composition selon l'une quelconque des revendications précédentes ; et
c.) facultativement laver et/ou rincer ledit site .

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US P6592990 B
- US 4515705 A
- US 4537706 A
- US 4537707 A
- US 4550862 A
- US 4561998 A
- US 4597898 A
- US 4968451 A
- US 5565145 A
- US 5929022 A
- US 6294514 A
- US 6376445 A
- US 20040204337 A1
- US 20040229769 A1
- US 20020077265 A1
- US 20040142841 A1
- US 20030216274 A1
- US 20040038851 A1
- US 20040065208 A1
- US 20050020476 A1
- US 3862058 A
- US 3948790 A
- US 3954632 A
- US 4062647 A
- US 5759990 A
- US 6494920 A
- WO 0218451 A
- WO 06007911 A1
- WO 06007899 A1
- US 5576282 A
- US 6306812 B1
- US 6326348 B1
- US 4430243 A
- US 5597936 A
- US 5595967 A
- WO 0032601 A
- US 6225464 B
- US 5879584 A
- US 5691297 A
- US 5574005 A
- US 5569645 A
- US 5565422 A
- US 5516448 A
- US 5489392 A
- US 5486303 A
- US 6869923 B1
- US 5574179 A
- WO 9510591 A
- US 20030108501 A1

### Non-patent literature cited in the description

- **L.J. Van Gemert.** TNO-CIVO Food Analysisi Institute, P.O. Box 360, 3700 AJ Zeist. IRL Press, 1990
- **Zhang, Z. ; Sun, G.** Mechanical Properties of Melamine-Formaldehyde microcapsules. *J. Microencapsulation,* 2001, vol. 18 (5), 593-602
- **A. Leo.** Comprehensive Medicinal Chemistry. Pergamon Press, 1990, vol. 4, 295